# EUROPEAN PATENT APPLICATION

(11) **EP 4 062 906 A1**
(43) Date of publication of application: **28.09.2022**
(21) Application number: 20888989.9
(22) Date of filing: 20.11.2020
(51) Int. Cl.: A61K 9/14, A61K 9/00, A61K 9/20, A61K 9/48, A61K 31/41, A61P 25/08

(54) **ORAL PHARMACEUTICAL COMPOSITION COMPRISING CARBAMATE COMPOUND AND PREPARATION METHOD THEREFOR**

(30) Priority: 22.11.2019 KR 20190151556
(71) Applicant: SK Biopharmaceuticals Co., Ltd., Seongnam-si, Gyeonggi-do 13494 (KR)
(72) Inventor: LEE, Ji Hye, Seongnam-si Gyeonggi-do 13494 (KR); CHOI, So Young, Seongnam-si Gyeonggi-do 13494 (KR)
(74) Representative: Vossius & Partner Patentanwälte Rechtsanwälte mbB
(86) International application number: PCT/KR2020/016427
(87) International publication number: WO 2021/101295

(57) **Abstract**

The present invention relates to an oral pharmaceutical composition comprising a carbamate compound of chemical formula 1, an isomer thereof, or a pharmaceutically acceptable salt, solvate, or hydrate thereof as an active ingredient, and a preparation method therefor.

## Description

### TECHNICAL FIELD

The present invention relates to a pharmaceutical composition for oral administration comprising a carbamate compound of the following Formula 1, an isomer thereof, or a pharmaceutically acceptable salt, solvate or hydrate thereof as an active ingredient, and a preparation method therefor: 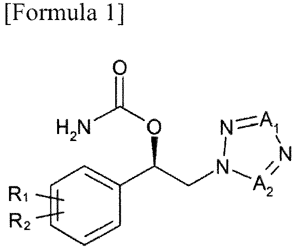 wherein,
R₁, R₂, A₁ and A₂ are the same as defined herein.

### BACKGROUND ART

The carbamate compound (carbamic acid aryl-2-tetrazolyl ethyl ester) represented by the following Formula 1 and the preparation method therefor are described in detail in International Patent Publication Nos. WO 2006/112685 A1, WO 2010/150946 A1 and WO 2011/046380 A2, which are incorporated herein by reference: 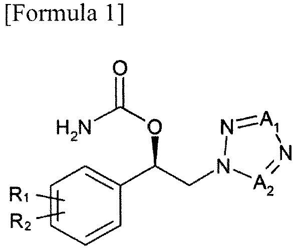 wherein R₁ and R₂ are each independently selected from the group consisting of hydrogen, halogen, C₁-C₈ perfluoroalkyl, C₁-C₈ alkyl, C₁-C₈ thioalkoxy and C₁-C₈ alkoxy; and one of A₁ and A₂ is CH, and the other is N.

A specific example of the carbamate compound of Formula 1 may be a carbamate compound of the following Formula 2 (carbamic acid (R)-1-(2-chlorophenyl)-2-tetrazol-2-yl-ethyl ester):

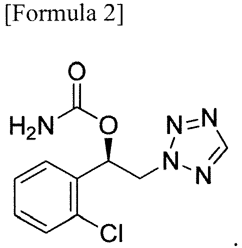

The carbamate compound of Formula 2 is known as an effective anti-epileptic drug used for central nervous system diseases, but studies about a specific formulation for oral administration for applying it to the human body have not been disclosed. In order for drugs to be applied to the human body, formulation design is essential. In order to be effective as drugs, specific formulations such as tablets, capsules, injections and ointments are required.

In the case of obtaining pharmacological activity by administering said compound, it is required that the effect must appear quickly, and it must be suitable to secure a uniform concentration of the active ingredient in the blood by repeating administration over an extended treatment period. In order to obtain a quick effect, an injection preparation may be appropriate, but there is a disadvantage in that use is limited due to the route of administration. As such, there is increased demand for developing a novel oral solid dosage form for achieving this purpose.

### DISCLOSURE OF INVENTION

### TECHNICAL PROBLEM

The solubility (1.8 to 2.0 mg/mL) of the carbamate compound of Formula 1 in the *in vivo* pH range (pH 1.2 to 6.8) does not limit the absorption of tablets having an active ingredient of 12.5 to 400 mg (BCS; Amidon, G.L. et al., Pharmaceutical research, 12: 413-420 (1995)). In addition, based on the above dose, the particle size of the compound is not expected to have a significant effect on uniformity of dosage units. Rather, micronization is not recommended for the purpose of uniformity of dosage units since it directly affects the flowability and stability of the active pharmaceutical ingredient, thereby affecting the content uniformity and content.

However, the present inventors have found that the dissolution rate of the carbamate compound of Formula 1 is variable despite its high solubility. The dissolution rate of the formulation is a necessary condition for rapid and consistent therapeutic effect, and quality control. In addition, if the dissolution rate is variable, it may cause problems in the quality control of the formulation. Therefore, the present inventors have conducted repeated studies to solve such problems.

Meanwhile, in general, micronization of particles is not adopted since it may directly affect the flowability and stability of drugs, thereby affecting the content uniformity and content of dosage forms. However, through repeated studies, the present inventors have conceived the dosage form which can consistently achieve an excellent dissolution rate by controlling the average particle size of the particles of the carbamate compound of Formula 1 included therein by micronization. That is, the present invention is surprising in that a person skilled in the art would not have easily conceived that the dissolution rate of the carbamate compound of Formula 1 can be adjusted by micronization.

Accordingly, an object of the present invention is the provision of a pharmaceutical composition for oral administration comprising a carbamate compound of Formula 1, an isomer thereof, or a pharmaceutically acceptable salt, solvate or hydrate thereof as an active ingredient, and a preparation method therefor.

### SOLUTION TO PROBLEM

According to one aspect of the present invention, there is provided a pharmaceutical composition for oral administration comprising particles of a carbamate compound of the following Formula 1, an isomer thereof, or a pharmaceutically acceptable salt, solvate or hydrate thereof as an active ingredient; and a pharmaceutically acceptable carrier; wherein a particle diameter d(0.9) of the active ingredient particles is less than 300 µm: 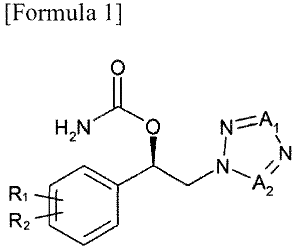 wherein,
R₁ and R₂ are each independently selected from the group consisting of hydrogen, halogen, C₁-C₈ perfluoroalkyl, C₁-C₈ alkyl, C₁-C₈ thioalkoxy and C₁-C₈ alkoxy; and
one of A₁ and A₂ is CH, and the other is N.

According to another aspect of the present invention, there is provided a method for preparing a pharmaceutical composition for oral administration comprising a step of mixing particles of the carbamate compound of the above Formula 1, an isomer thereof, or a pharmaceutically acceptable salt, solvate or hydrate thereof as an active ingredient with a pharmaceutically acceptable carrier, and tableting; wherein a particle diameter d(0.9) of the active ingredient particles is less than 300 µm.

### EFFECTS OF INVENTION

According to the present invention, it is possible to provide an oral solid dosage form that shows rapid and consistent therapeutic effects by accomplishing excellent disintegration and a rapid dissolution.

### BRIEF DESCRIPTION OF DRAWINGS

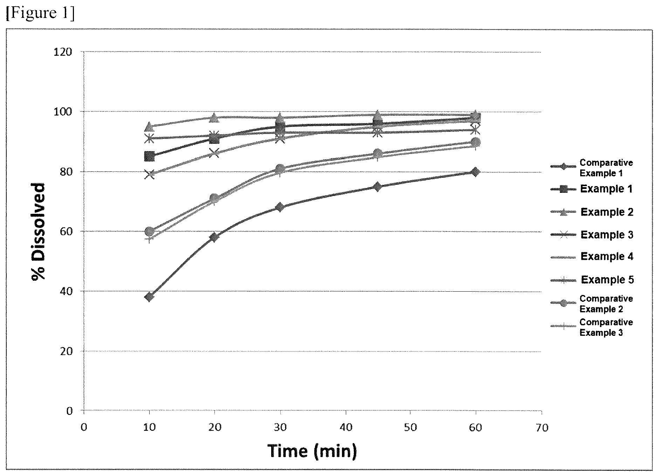

Figure 1 is a graph showing the dissolution test results of Examples 1 to 5 and Comparative Examples 1 to 3.

### MODE FOR THE INVENTION

The present invention is described in detail hereinafter.

All technical terms used in the present invention, unless otherwise defined, have the same meaning as commonly understood by a person skilled in the art of the present invention. In addition, although preferred methods and samples are described herein, similar or equivalent ones are also included within the scope of the present invention.

It should be understood that all numerical designations-for example, pH, temperature, time, concentration, d(0.9) and d(0.5)-used throughout the instant specification may be modified by the term "about" in all instances. When the term "about" is used in the description of the present invention, it may mean ±10%, ±5%, ±2% or ±1 % in reference to a percentage. In one embodiment, it may mean ±5%, ±2% or ±1%. For example, "about 5" is meant to include any value between 4.5 and 5.5, between 4.75 and 5.25, between 4.9 and 5.1, or between 4.95 and 5.05.

As used herein, the term "particle" refers to individual drug substance particles, whether the particles exist alone or aggregated. That is, the pharmaceutical composition of the present invention comprising the carbamate compound of Formula 1 may contain aggregates having a particle diameter d(0.9) of 300 µm or more. However, in the case in which the particle diameter d(0.9) of the main drug particles constituting the aggregate is less than 300 µm, 250 µm or less, 200 µm or less, 150 µm or less, 130 µm or less, or 100 µm or less, it is considered that the aggregate itself satisfies the particle size constraints defined herein, and the composition is within the scope of the present invention.

Herein, with respect to the particles of the carbamate compound of Formula 1, reference to particle size such as particle diameter d(0.9) and particle diameter d(0.5) means that the average of particles of the carbamate compound of Formula 1 in the sample has an estimated volume less than or equal to the volume calculated for spherical particles with a diameter equal to a given diameter, based on the assumption that the shape of the particles is spherical. The particle size distribution is well known to those skilled in the art and can be measured by a laser light scattering technique such as that disclosed and discussed below. In one embodiment of the present invention, the particle size of the carbamate compound of Formula 1 was measured by the use of a Malvern particle size analyzer.

As used herein, "d(0.9)" means that 90% of the particle volume has a diameter in a specific diameter d range. Specifically, it means that the particle diameter d(0.9) of the point where the cumulative frequency of the volume distribution reaches 90% by accumulating from the particle of the smaller particle diameter is within the range of the specific diameter d.

As used herein, "d(0.5)" means that 50% of the particle volume has a diameter in a specific diameter d range. Specifically, it means that the particle diameter d(0.5) of the point where the cumulative frequency of the volume distribution reaches 50% by accumulating from the particle of the smaller particle diameter is within the range of the specific diameter d.

The present invention provides an oral pharmaceutical composition containing the carbamate compound of Formula 1, an isomer thereof, or a pharmaceutically acceptable salt, solvate or hydrate thereof, and a preparation method therefor.

All specific details related to the present invention described below can be clearly applied to the pharmaceutical composition for oral administration of the present invention as well as the preparation method therefor. 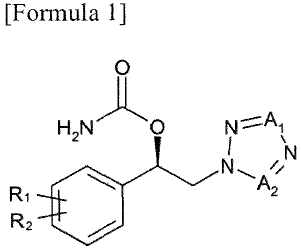 wherein,
R₁ and R₂ are each independently selected from the group consisting of hydrogen, halogen, C₁-C₈ perfluoroalkyl, C₁-C₈ alkyl, C₁-C₈ thioalkoxy and C₁-C₈ alkoxy; and
one of A₁ and A₂ is CH, and the other is N.

In the present invention, the carbamate compound of Formula 1, an isomer thereof, or a pharmaceutically acceptable salt, solvate or hydrate thereof may have a particle size distribution in which the particle diameter d(0.9) of the point where the cumulative frequency of the volume distribution reaches 90% by accumulating from the particle of the smaller particle diameter is less than 300 µm, and more specifically, may have a particle diameter distribution in which the particle diameter d(0.9) is 250 µm or less, 200 µm or less, 150 µm or less, 130 µm or less, or 100 µm or less. The lower limit of the particle diameter d(0.9) is not specifically limited, and may be, for example, greater than 0 µm, 30 µm or more, or 50 µm or more, but is not limited thereto.

In one embodiment of the present invention, in the carbamate compound of Formula 1, an isomer thereof, or a pharmaceutically acceptable salt, solvate or hydrate thereof, the particle diameter d(0.5) of the point where the cumulative frequency of the volume distribution reaches 50% by accumulating from the particle of the smaller particle diameter may be in the range of 5 to 80 µm, and more specifically, the particle diameter d(0.5) may be in the range of 7 to 70 µm, 10 to 60 µm, or 15 to 50 µm.

The pharmaceutical composition of the present invention may be any solid dosage form known in this technical field. Specifically, the solid dosage form may be in the form of tablets or capsules, and more specifically, may be in the form of compressed tablets, multi-compressed tablets, sugar-coated tablets, film-coated tablets, hard capsules or soft capsules. Preferably, the solid dosage form may be in the form of tablets, particularly in the form of compressed tablets or film-coated tablets, but is not limited thereto.

The oral solid dosage form according to the present invention preferably shows the following dissolution criteria when tested for *in vitro* dissolution. That is, the oral solid dosage form shows dissolution properties in which an amount of the drug exceeding 81% by weight is dissolved within 30 minutes. Preferably, the oral solid dosage form shows dissolution properties in which 85% by weight or more, more preferably 90% by weight or more, and even more preferably 91% by weight or more of the drug is dissolved within 30 minutes.

Conventionally, the results of dissolution tests are established as an average over a given number-usually six (6) administration forms (e.g., tablets, capsules, suspensions or other administration forms). Dissolution tests are typically performed in aqueous medium buffered to the pH range observed in the gastrointestinal tract (pH 1 to 7.4) and adjusted to 37°C (±1°C) to maintain physiologically relevant conditions. When the administration form being tested is a tablet, a paddle rotating at 50 to 75 rpm is typically used to test the dissolution rate of the tablet. The amount of dissolved carbamate compound of Formula 1 can be determined routinely by HPLC. The dissolution test serves as a quality control tool.

In one embodiment, the pharmaceutical composition for oral administration of the present invention may comprise the active ingredient (i.e., the carbamate compound of Formula 1, an isomer thereof, or a pharmaceutically acceptable salt, solvate or hydrate thereof) in a dose-range of 5 mg to 400 mg. More specifically, the active ingredient may be comprised in a dose of 12.5 mg, 25 mg, 50 mg, 100 mg, 150 mg or 200 mg.

In one embodiment, when the pharmaceutical composition for oral administration of the present invention is a tablet, the tablet may be prepared by direct mixing of the carbamate compound of Formula 1, an isomer thereof, or a pharmaceutically acceptable salt, solvate or hydrate thereof with a pharmaceutically acceptable carrier, or by dry/wet granulation tableting.

In one embodiment, the pharmaceutically acceptable carrier may be selected from the group consisting of a diluent, a disintegrant, a lubricant and any combination thereof, but is not limited thereto.

In one embodiment, the pharmaceutically acceptable carrier may further comprise a surfactant.

In one embodiment, the diluent may be one or more selected from the group consisting of corn starch, pre-gelatinized starch, potato starch, wheat flour starch, glutinous rice starch, sweet potato starch, tapioca starch, rice starch, beeswax corn starch, sucrose, anhydrous lactose, lactose hydrate, mannitol, sorbitol, xylitol, lactitol, maltitol, erythritol, synthetic aluminum silicate, hydroxypropyl starch, microcrystalline cellulose and crystalline cellulose. Specifically, the diluent may be one or more selected from the group consisting of lactose hydrate, synthetic aluminum silicate, hydroxypropyl starch, microcrystalline cellulose and crystalline cellulose. More specifically, the diluent may be one or more selected from the group consisting of lactose hydrate and microcrystalline cellulose, but is not limited thereto. In addition, in one embodiment, microcrystalline cellulose may be used as the first diluent, and lactose may be used as the second diluent.

In one embodiment, the disintegrant may be one or more selected from the group consisting of low-substituted hydroxypropyl cellulose, microcrystalline cellulose, starch, anhydrous lactose, lactose hydrate, sodium starch glycolate, crospovidone, carboxymethyl cellulose and a pharmaceutically acceptable salt thereof, hydroxypropyl cellulose, corn starch, and croscarmellose and a pharmaceutically acceptable salt thereof. Specifically, the disintegrant may be one or more selected from the group consisting of sodium starch glycolate, crospovidone, carboxymethylcellulose and a pharmaceutically acceptable salt thereof, hydroxypropyl cellulose, corn starch, and croscarmellose and a pharmaceutically acceptable salt thereof. More specifically, the disintegrant may be sodium starch glycolate, but is not limited thereto.

In one embodiment, the lubricant may be one or more selected from the group consisting of silicon dioxide, colloidal anhydrous silica, magnesium trisilicate, tribasic calcium phosphate, calcium silicate, magnesium silicate, colloidal silicon dioxide, powdered cellulose, starch, magnesium stearate, talc, light anhydrous silicic acid, sodium stearyl fumarate, polyethylene glycol, mineral oil, hydrogenated vegetable oil, zinc stearate and stearic acid. More specifically, the lubricant may be colloidal silicon dioxide, magnesium stearate or a combination thereof, but is not limited thereto.

In one embodiment, the surfactant may be one or more selected from the group consisting of polysorbate 80, oleoyl macrogol glyceride, linoleoyl, caprylocaproyl macrogol glyceride, polyoxylglyceride, hydroxypropyl methylcellulose (HPMC), hydroxypropyl cellulose (HPC), sodium carboxymethylcellulose, polyvinylpyrrolidone, sodium lauryl sulfate, sodium oleate and sodium dioctyl sulfosuccinate. Specifically, the surfactant may be one or more selected from the group consisting of sodium lauryl sulfate, sodium oleate, and sodium dioctyl sulfosuccinate. More specifically, the surfactant may be sodium lauryl sulfate, but is not limited thereto.

In one embodiment, the pharmaceutical composition for oral administration of the present invention may comprise the following components when it is prepared by a direct compression method:
5 to 35% by weight of the active ingredient, 55 to 90% by weight of the diluent, 2 to 6% by weight of the disintegrant and 0.1 to 4% by weight of the lubricant, based on the total weight of the pharmaceutical composition.

In another embodiment, the pharmaceutical composition for oral administration of the present invention may comprise the following components when it is prepared by a direct compression method:
10 to 30% by weight of the active ingredient, 65 to 85% by weight of the diluent, 3 to 5% by weight of the disintegrant and 0.3 to 1% by weight of the lubricant, based on the total weight of the pharmaceutical composition.

In addition to the above-mentioned components, the pharmaceutical composition of the present invention may further comprise other components such as a binder, a film coating agent, a colorant, a fragrance, a sweetener, a flavoring agent and a preservative within a range that does not impair the purpose of the present invention.

In one embodiment, when the pharmaceutical composition according to the present invention is in the form of a film-coated tablet, the pharmaceutical composition may comprise a film coating agent. Typically, the film coating agent may be comprised in an amount of 2 to 4% by weight based on the total weight of the pharmaceutical composition, and which includes a film-forming agent, a plasticizer, a lubricant and optionally one or more pigments. As described below, the film-coated tablet may be prepared by additionally carrying out a coating step after direct compression. As the film coating agent, a conventional film coating agent such as Opadry may be used.

In the present invention, a preferred active ingredient is a carbamate compound of the following Formula 2, an isomer thereof, or a pharmaceutically acceptable salt, solvate or hydrate thereof:

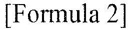

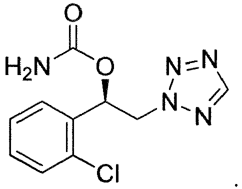

In addition, the present invention provides a method for preparing a pharmaceutical composition for oral administration comprising a step of mixing particles of the carbamate compound of Formula 1, an isomer thereof, or a pharmaceutically acceptable salt, solvate or hydrate thereof as an active ingredient with a pharmaceutically acceptable carrier, and tableting; wherein the average particle size of the lower 90% of the active ingredient particles (d(0.9)) is less than 300 µm.

The tableting may be carried out according to any method known in this technical field, and preferably may be direct compression.

The pharmaceutical composition for oral administration prepared by the above method may be the shape-for example, round, oval, oblong, rectangular, cylindrical or other suitable shape. In addition, the size may be changed according to the concentration of the active ingredient.

In one embodiment, when the pharmaceutical composition according to the present invention is in the form of a coated tablet, the step of coating after tableting may be further comprised. Specifically, the coated tablet may be a film-coated tablet. Typically, the film coating agent may be comprised in an amount of 2% by weight to 4% by weight based on the total weight of the pharmaceutical composition. The film coating agent may include a film-forming agent, a plasticizer, a lubricant and optionally one or more pigments. As the film coating agent, a conventional film coating agent such as Opadry may be used.

The pharmaceutical composition provided according to the present invention can be used for preventing or treating diseases of the central nervous system.

In one embodiment, the central nervous system disease may be selected from anxiety, depression, convulsion, epilepsy, migraine, bipolar disorder, drug abuse, smoking, attention deficit hyperactivity disorder (ADHD), obesity, sleep disorders, stroke, neuropathic pain, cognitive disorders, neurodegeneration and muscle spasm, but is not limited thereto.

As used herein, the terms "prevent," "preventing" and "prevention" refer to reducing or eliminating the likelihood of a disease.

As used herein, the terms "treat," "treating" and "treatment" refer to eliminating a disease and/or its accompanying symptoms altogether or in part.

Hereinafter, the present invention will be explained in more detail through working examples. However, the following working examples are only intended to illustrate one or more embodiments and are not intended to limit the scope of the invention.

### Preparation Example: Synthesis of Test Compound (carbamic acid (R)-1-(2-chlorophenyl)-2-tetrazol-2-yl-ethyl ester)

Carbamic acid (R)-1-(2-chlorophenyl)-2-tetrazol-2-yl-ethyl ester (the carbamate compound of Formula 2) was prepared according to the method described in Preparation Example 50 of PCT Publication No. WO 2010/150946.

### Examples 1 to 5 and Comparative Examples 1 to 3: Preparation of oral dosage forms comprising Test Compound by direct compression

According to the compositions and contents represented in Table 1, the components were mixed and tableted (direct compression). Thereafter, the tablets were film-coated with a coating agent according to a conventional coating method for tablets.

**[Table 1]**

| Item | | Comparative Example 1 | Example 1 | Example 2 | Example 3 | Example 4 | Example 5 | Comparative Example 2 | Comparative Example 3 |
|---|---|---|---|---|---|---|---|---|---|
| Active ingredient | Carbamate compound of Formula 2 (mg) | 200.0 | 200.0 | 200.0 | 50.0 | 50.0 | 50.0 | 200.0 | 200.0 |
| | Particle size d(0.9) | 460 µm | 166 µm | 50 µm | 112 µm | 206 µm | 99 µm | 300 µm | 300 µm |
| | Particle size d(0.5) | 220 µm | 37 µm | 18 µm | 34 µm | 48 µm | 24 µm | - | - |
| Diluent | Microcrystalline cellulose (mg) | 345.20 | 345.20 | 345.20 | 172.60 | 172.60 | 172.60 | 337.20 | 345.20 |
| | Lactose hydrate (mg) | 217.36 | 217.36 | 217.36 | 158.68 | 158.68 | 158.68 | 217.36 | 217.36 |
| Disintegrant | Sodium starch glycolate (mg) | 30.48 | 30.48 | 30.48 | 15.24 | 15.24 | 15.24 | 30.48 | 30.48 |
| Lubricant | Colloidal silicon dioxide (mg) | 2.24 | 2.24 | 2.24 | 1.12 | 1.12 | 1.12 | 2.24 | 2.24 |
| | Magnesium stearate (mg) | 4.72 | 4.72 | 4.72 | 2.36 | 2.36 | 2.36 | 4.72 | 4.72 |
| Surfactant | Sodium lauryl sulfate (mg) | - | - | - | - | - | - | 8 | - |
| Total weight (mg) | | 800 | 800 | 800 | 400 | 400 | 400 | 800 | 800 |

### Experimental Example: Dissolution test

A dissolution test was performed on the solid dosage forms prepared in Examples 1 to 5 and Comparative Examples 1 to 3 according to the US Pharmacopoeia under the following conditions.

### <Dissolution conditions>

Dissolution media: 0.01 N hydrochloric acid aqueous solution, 900 mL
Device: Apparatus II (paddle method), 75 rpm
Temperature: 37°C

The results of dissolution rate are represented in Table 2 and Figure 1.

After 10, 20, 30, 45 and 60 minutes from the start of the test, the samples were taken out and analyzed for Test Compound by HPLC at 215 nm.

**[Table 2]**

| Time (min.) | Dissolved active ingredient (% by weight) | | | | | | | |
|---|---|---|---|---|---|---|---|---|
| | Comparative Example 1 | Example 1 | Example 2 | Example 3 | Example 4 | Example 5 | Comparative Example 2 | Comparative Example 3 |
| 0 | 0 | 0 | 0 | 0 | 0 | 0 | 0 | 0 |
| 10 | 38 | 85 | 95 | 91 | 79 | 91 | 60 | 57 |
| 20 | 58 | 91 | 98 | 92 | 86 | 92 | 71 | 70 |
| 30 | 68 | 95 | 98 | 93 | 91 | 93 | 81 | 80 |
| 45 | 75 | 96 | 99 | 95 | 95 | 93 | 86 | 85 |
| 60 | 80 | 98 | 99 | 96 | 97 | 94 | 90 | 89 |

## Claims

1. A pharmaceutical composition for oral administration comprising particles of a carbamate compound of the following Formula 1, an isomer thereof, or a pharmaceutically acceptable salt, solvate or hydrate thereof as an active ingredient; and a pharmaceutically acceptable carrier; wherein a particle diameter d(0.9) of the active ingredient particles is less than 300 µm: 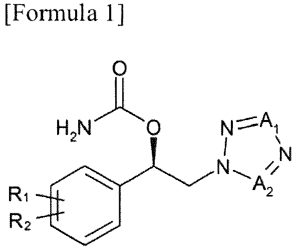 wherein,
R₁ and R₂ are each independently selected from the group consisting of hydrogen, halogen, C₁-C₈perfluoroalkyl, C₁-C₈ alkyl, C₁-C₈ thioalkoxy and C₁-C₈ alkoxy; and
one of A₁ and A₂ is CH, and the other is N.

2. The pharmaceutical composition for oral administration according to Claim 1, wherein the particle diameter d(0.9) of the active ingredient particles is 250 µm or less.

3. The pharmaceutical composition for oral administration according to Claim 1, wherein the particle diameter d(0.9) of the active ingredient particles is 150 µm or less.

4. The pharmaceutical composition for oral administration according to any one of Claims 1 to 3, wherein the active ingredient is comprised in an amount of 5 mg to 400 mg.

5. The pharmaceutical composition for oral administration according to any one of Claims 1 to 4, wherein the pharmaceutically acceptable carrier is one or more selected from the group consisting of a diluent, a disintegrant and a lubricant.

6. The pharmaceutical composition for oral administration according to Claim 5, which further comprises a surfactant.

7. The pharmaceutical composition for oral administration according to Claim 5 or 6, wherein the diluent is one or more selected from the group consisting of corn starch, pre-gelatinized starch, potato starch, wheat flour starch, glutinous rice starch, sweet potato starch, tapioca starch, rice starch, beeswax corn starch, sucrose, anhydrous lactose, lactose hydrate, mannitol, sorbitol, xylitol, lactitol, maltitol, erythritol, synthetic aluminum silicate, hydroxypropyl starch, microcrystalline cellulose and crystalline cellulose.

8. The pharmaceutical composition for oral administration according to any one of Claims 5 to 7, wherein the disintegrant is one or more selected from the group consisting of low-substituted hydroxypropyl cellulose, microcrystalline cellulose, starch, anhydrous lactose, lactose hydrate, sodium starch glycolate, crospovidone, carboxymethyl cellulose and a pharmaceutically acceptable salt thereof, hydroxypropyl cellulose, corn starch, and croscarmellose and a pharmaceutically acceptable salt thereof.

9. The pharmaceutical composition for oral administration according to any one of Claims 5 to 8, wherein the lubricant is one or more selected from the group consisting of silicon dioxide, colloidal anhydrous silica, magnesium trisilicate, tribasic calcium phosphate, calcium silicate, magnesium silicate, colloidal silicon dioxide, powdered cellulose, starch, magnesium stearate, talc, light anhydrous silicic acid, sodium stearyl fumarate, polyethylene glycol, mineral oil, hydrogenated vegetable oil, zinc stearate and stearic acid.

10. The pharmaceutical composition for oral administration according to any one of Claims 5 to 9, which comprises 5 to 35% by weight of the active ingredient, 55 to 90% by weight of the diluent, 2 to 6% by weight of the disintegrant and 0.1 to 4% by weight of the lubricant, based on the total weight of the pharmaceutical composition.

11. The pharmaceutical composition for oral administration according to any one of Claims 1 to 10, wherein the active ingredient is a carbamate compound of the following Formula 2, an isomer thereof, or a pharmaceutically acceptable salt, solvate or hydrate thereof:

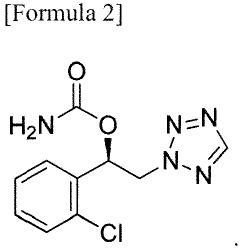

12. The pharmaceutical composition for oral administration according to any one of Claims 1 to 11, which is in a form of compressed tablet, multi-compressed tablet, sugar-coated tablet, film-coated tablet, hard capsule or soft capsule.

13. A method for preparing a pharmaceutical composition for oral administration comprising a step of mixing particles of a carbamate compound of the following Formula 1, an isomer thereof, or a pharmaceutically acceptable salt, solvate or hydrate thereof as an active ingredient with a pharmaceutically acceptable carrier, and tableting;
wherein a particle diameter d(0.9) of the active ingredient particles is less than 300 µm: 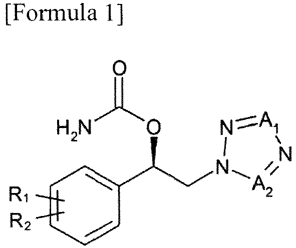 wherein,
R₁ and R₂ are each independently selected from the group consisting of hydrogen, halogen, C₁-C₈ perfluoroalkyl, C₁-C₈ alkyl, C₁-C₈ thioalkoxy and C₁-C₈ alkoxy; and
one of A₁ and A₂ is CH, and the other is N.

14. The method for preparing a pharmaceutical composition for oral administration according to Claim 13, wherein the mixing of the active ingredient with the pharmaceutically acceptable carrier is carried out by direct compression.

15. The method for preparing a pharmaceutical composition for oral administration according to Claim 13 or 14, wherein the particle diameter d(0.9) of the active ingredient particles is 250 µm or less.

16. The method for preparing a pharmaceutical composition for oral administration according to any one of Claims 13 to 15, wherein the pharmaceutical composition comprises the active ingredient in an amount of 5 mg to 400 mg.

17. The method for preparing a pharmaceutical composition for oral administration according to any one of Claims 13 to 16, wherein the pharmaceutically acceptable carrier is one or more selected from the group consisting of a diluent, a disintegrant and a lubricant.

18. The method for preparing a pharmaceutical composition for oral administration according to Claim 17, wherein the pharmaceutically acceptable carrier further comprises a surfactant.

19. The method for preparing a pharmaceutical composition for oral administration according to any one of Claims 13 to 18, wherein the active ingredient is a carbamate compound of the following Formula 2, an isomer thereof, or a pharmaceutically acceptable salt, solvate or hydrate thereof:

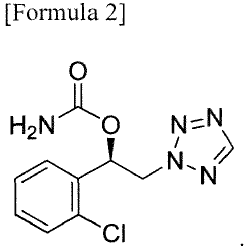

20. The method for preparing a pharmaceutical composition for oral administration according to any one of Claims 13 to 18, wherein the pharmaceutical composition is in a form of compressed tablet, multi-compressed tablet, sugar-coated tablet, film-coated tablet, hard capsule or soft capsule.
